# EUROPÄISCHE PATENTSCHRIFT

(11) **EP 3 329 877 B1**
(45) Veröffentlichungstag und Bekanntmachung des Hinweises auf die Patenterteilung: **25.11.2020**
(21) Anmeldenummer: 17197308.4
(22) Anmeldetag: 19.10.2017
(51) Int. Cl.: B25J 9/16, A61B 34/20, A61B 34/30

(54) **BERECHNEN EINES KALIBRIERUNGSPARAMETERS EINES ROBOTERWERKZEUGS**
CALCULATION OF A CALIBRATION PARAMETER OF A ROBOT TOOL
PROCÉDÉ DE CALCUL D'UN PARAMÈTRE D'ÉTALONNAGE D'UN OUTIL ROBOTISÉ

(30) Priorität: 30.11.2016 DE 102016223841
(43) Veröffentlichungstag der Anmeldung: 06.06.2018
(73) Patentinhaber: Siemens Healthcare GmbH, 91052 Erlangen (DE)
(72) Erfinder: Gulhar, Abhinav, 91054 Erlangen (DE); Mewes, Philip, 90439 Nürnberg (DE)

(56) Entgegenhaltungen:
- EP-A1- 1 854 425
- EP-A1- 3 558 599
- WO-A1-2015/197100
- WO-A2-2007/136768
- DE-A1-102014 219 581
- US-A1- 2009 118 864
- US-A1- 2009 192 524
- US-A1- 2013 274 921

## Beschreibung

Roboter werden in medizinischen Anwendungen insbesondere dort eingesetzt, wo eine hohe Präzision oder eine hohe Ausdauer notwendig ist. Hierzu gehören Biopsien, Hochfrequenzablationen oder die Platzierung von orthopädischen Schrauben.

Die medizinische Anwendung von Robotern wird häufig durch bildgebende Geräte wie Magnetresonanztomographen, Computertomographen, Ultraschallgeräte oder Röntgengeräte unterstützt. Diese werden dazu verwendet, innere Strukturen des menschlichen Körpers darzustellen, um die medizinischen Werkzeuge korrekt zu platzieren.

Um Verletzungen von gesundem Gewebe, Knochen oder Organen außerhalb des Operationsbereichs zu vermeiden, müssen sehr genaue Informationen über die Position und Orientierung des Roboterwerkzeugs vorliegen. Daher müssen Roboterwerkzeuge kalibriert werden.

Es ist bekannt, Roboter anschließend an ihre Produktion zu kalibrieren. Hierbei kann aber höchstens die Position der Werkzeugaufnahme kalibriert werden, da Roboterwerkzeuge meist unabhängig vom Roboter bereitgestellt werden und eine andere Nutzungsdauer haben. Für medizinische Anwendungen ist aber gerade eine Kalibrierung des Roboterwerkzeugs notwendig, insbesondere des aktiven Punktes des Roboterwerkzeugs, der auch als Werkzeugmittelpunkt (ein englischer Fachbegriff ist "tool center point", kurz "TCP") bezeichnet wird.

Daher muss jedes Roboterwerkzeug separat zusammen mit dem Roboter kalibriert werden. Weiterhin ist es aufgrund von Abnutzung des Roboterwerkzeugs notwendig, eine Kalibrierung in gewissen Zeitabständen auszuführen.

Es ist bekannt, eine separate Kalibrierungseinheit zu verwenden, um den Werkzeugmittelpunkt eines Roboterwerkzeugs zu kalibrieren. Dabei umfassen derartige Kalibrierungseinheiten Positionsbestimmungseinheiten beispielsweise Ultraschallsensoren, Laserinterferometrie oder Lasertriangulation. Eine solche Methode ist beispielsweise aus der Druckschrift WO 2007/136768 A2 bekannt, hierbei wird die Kalibrierung mittels eines Vergleichs eines optischen Trackings von Markierungen und Parametern des Roboters durchgeführt. Diese separaten Kalibrierungseinheiten sind aber teuer und schlecht zu transportieren.

Weiterhin ist auch die Verwendung separaten optischen Kalibrierungseinheiten bekannt. Die Druckschriften EP 1854425 A1, US 2009/118864 A1 sowie US 2009/192524 A1 verwenden jeweils optische Kameras, um bildgestützte Positionen eines Werkzeugs bzw. eines Werkzeugmittelpunktes zu bestimmen.

Weiterhin ist bekannt, die Kalibrierung durchzuführen, indem durch Steuerung einer Bedienperson mit dem Werkzeugmittelpunkt mehrere vorgegebene Punkte berührt werden. Diese Methode ist aber zeitaufwändig und abhängig von den Fertigkeiten und der Präzision der Bedienperson.

Es ist daher die Aufgabe der vorliegenden Erfindung, eine Methode zur kostengünstigen und präzisen Kalibrierung des Werkzeugmittelpunkts eines Roboterwerkzeugs bereitzustellen.

Die Aufgabe wird gelöst durch ein Verfahren nach Anspruch 1, durch eine Kalibrierungseinheit nach Anspruch 11, durch ein Computerprogrammprodukt nach Anspruch 13, durch ein computerlesbares Speichermedium nach Anspruch 14 sowie durch einen Roboter nach Anspruch 15.

Nachstehend wird die erfindungsgemäße Lösung der Aufgabe in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert darauf, dass ein Bilddatensatz einer medizinischen Bildgebung eines Bildvolumens mittels einer ersten Schnittstelle empfangen wird, wobei das Bildvolumen einen Teil des Roboterwerkzeugs umfasst, und wobei das Roboterwerkzeug an einem Roboter befestigt ist. Hierbei ist die medizinische Bildgebung eine Aufnahme des Bildvolumens mit einem Magnetresonanztomographen, einem Computertomographen, einem Ultraschallgerät und/oder einem Röntgengerät. Weiterhin wird ein Roboterdatensatz mittels einer zweiten Schnittstelle empfangen, wobei der Roboterdatensatz eine Position wenigstens einer beweglichen Achse des Roboters während der Aufnahme des Bilddatensatzes umfasst. Weiterhin wird die Position und/oder Orientierung einer Markierung im Bilddatensatz mittels einer Recheneinheit bestimmt, wobei das Roboterwerkzeug die Markierung umfasst. Weiterhin wird eine bildgestützte Position und/oder Orientierung des Werkzeugmittelpunkts des Roboterwerkzeugs durch Transformieren der Position und/oder Orientierung der Markierung mittels der Recheneinheit berechnet. Weiterhin wird der Kalibrierungsparameter basierend auf dem Roboterdatensatz sowie auf der bildgestützten Position und/oder Orientierung des Werkzeugmittelpunkts mittels der Recheneinheit berechnet.

Die Erfinder haben erkannt, dass mittels eines Bilddatensatzes einer medizinischen Bildgebung der Kalibrierungsparameter besonders kostengünstig berechnet werden kann, da bei einer medizinischen Anwendung des Roboters in der Regel immer ein bildgebendes Gerät vorhanden ist und bei vielen Anwendungen des Roboters ein medizinischer Bilddatensatz zur Planung und/oder Überwachung eingesetzt wird. Daher ist neben dem bildgebenden Gerät keine zusätzliche bildbasierte Kalibrierungseinheit notwendig. Weiterhin kann eine Kalibrierung des Werkzeugmittelpunkts schnell und effizient ohne Umbau erfolgen, insbesondere also auch nach jedem Wechsel des Roboterwerkzeugs, insbesondere auch vor jedem Einsatz des Roboterwerkzeugs. Damit können durch den Wechsel oder den Verschleiß des Roboterwerkzeugs bedingte Abweichungen in die Kalibrierung mit einbezogen werden. Weiterhin haben medizinische Bilddaten eine hohe Auflösung, so dass der Kalibrierungsparameter besonders präzise berechnet werden kann.

Nach einem weiteren Aspekt der Erfindung umfasst der Bilddatensatz eine zweidimensionale Röntgenprojektion des Bildvolumens. Die Erfinder haben erkannt, dass zweidimensionale Röntgenprojektionen besonders schnell und kostengünstig aufgezeichnet werden können. Weiterhin können Röntgenbilddaten, insbesondere also zweidimensionale Röntgenprojektionen, Objekte oder Merkmale darstellen, die einer Bildgebung mit sichtbarem Licht nicht zugänglich sind.

Nach einem weiteren Aspekt der Erfindung umfasst der Bilddatensatz ein aus mehreren zweidimensionalen Röntgenprojektionen des Bildvolumens rekonstruiertes dreidimensionales Volumenbild. Die mehreren Röntgenprojektionen des Bildvolumens können insbesondere bezüglich zweier unterschiedlicher Projektionsrichtungen aufgenommen sein. Die Erfinder haben erkannt, dass in einem dreidimensionalem Volumenbild insbesondere auch dreidimensionale Bildinformationen über das Roboterwerkzeug vorliegen, und somit dreidimensionale Daten betreffend die Position und/oder Orientierung der Markierung. Damit kann der Kalibrierungsparameter besonders präzise berechnet werden.

Nach einem weiteren Aspekt der Erfindung ist das dreidimensionale Volumenbild aus zweidimensionalen radiographischen Röntgenprojektionen des Bildvolumens rekonstruiert. Dabei bezeichnen radiographische Röntgenprojektionen solche Röntgenprojektionen, die nicht mittels eines Computertomographens aufgenommen wurden. Die Erfinder haben erkannt, dass radiographische Röntgenprojektionen genügen, um ein dreidimensionales Volumenbild zur präzisen Kalibrierung eines Roboterwerkzeugs zu rekonstruieren. Gleichzeitig sind radiographische Röntgenprojektionen günstiger und schneller aufzunehmen als eine Computertomographie. Weiterhin umschließen Geräte zur Aufnahme von radiographische Röntgenprojektionen den Untersuchungsbereich nicht vollständig und ermöglichen daher einen einfacheren Zugang des Roboters zum Untersuchungsbereich.

Nach einem weiteren Aspekt der Erfindung ist die Struktur des Roboterwerkzeugs im Bilddatensatz dargestellt, und die Markierung ist als Struktur des Roboterwerkzeugs ausgebildet. Die Erfinder haben erkannt, dass die im Bilddatensatz dargestellte Struktur des Roboterwerkzeugs ausreicht, um die Position und/oder Orientierung des Roboterwerkzeugs und damit des Werkzeugmittelpunkts zu bestimmen. Damit kann der Kalibrierungsparameter besonders günstig berechnet werden, da keine Modifikation des Roboterwerkzeugs spezifisch für das Kalibrierungsverfahren notwendig ist.

Nach einem weiteren möglichen Aspekt der Erfindung kann die Struktur des Roboterwerkzeugs insbesondere eine innere Struktur des Roboterwerkzeugs betreffen. Die Erfinder haben erkannt, dass es durch die Verwendung der inneren Struktur als Markierung besonders präzise möglich ist, die Position und/oder Orientierung der Markierung zu bestimmen. Die Position und/oder Orientierung der Markierung kann insbesondere besonders präzise bestimmt werden, wenn die Oberfläche des Roboterwerkzeugs eine Symmetrie aufweist, die innere Struktur des Roboterwerkzeugs diese Symmetrie aber bricht. Weiterhin kann die Position und/oder Orientierung der Markierung insbesondere dann präzise bestimmt werden, wenn die innere Struktur einen hohen Kontrast im Bilddatensatz aufweist.

Nach einem weiteren Aspekt der Erfindung erfolgt das erste Berechnen der bildgestützten Position und/oder Orientierung des Werkzeugmittelpunkts durch Registrieren des Bilddatensatzes mit einem dreidimensionalen Modell des Roboterwerkzeugs. Die Erfinder haben erkannt, dass durch die Registrierung die bildgestützten Position und/oder Orientierung des Werkzeugmittelpunkts basierend auf der Position von wenigen markanten Punkten bestimmt werden kann. Insbesondere ist es nicht notwendig, die Position des Werkzeugmittelpunkts direkt im Bilddatensatz zu bestimmen. Zudem kann durch die Registrierung mit dem dreidimensionalen Modell des Roboterwerkzeugs besonders gut der Einfluss der Abnutzung auf die Position und/oder Orientierung des Werkzeugmittelpunkts erfasst werden. Weiterhin ist es durch die Registrierung möglich, produktionsbedingte Abweichungen des Roboterwerkzeugs von der idealen Form festzustellen und bei der Bestimmung der Position und/oder Orientierung des Werkzeugmittelpunkts zu berücksichtigen.

Nach einem weiteren Aspekt der Erfindung umfasst die Markierung eine Mehrzahl von Markierungselementen, wobei die Markierungselemente außen am Roboterwerkzeug und/oder innerhalb des Roboterwerkzeugs angeordnet sind. Die Erfinder haben erkannt, dass die dedizierten Markierungselemente so ausgebildet sein können, dass sie besonders gut im Bilddatensatz darstellbar sind. Dadurch ist die Bestimmung der bildgestützten Position und/oder Orientierung des Roboterwerkzeugs einerseits besonders einfach, andererseits aber auch sehr präzise möglich. Die Markierungselemente können insbesondere vom Roboterwerkzeug abnehmbar ausgebildet sein. In der abnehmbaren Ausführung ist vorteilhaft, wenn die Markierungselemente den Einsatz des Roboterwerkzeugs beeinflussen.

Erfindungsgemäß basiert das zweite Berechnen derart auf dem Roboterdatensatz, dass aus dem Roboterdatensatz eine prognostizierte Position und/oder Orientierung des Werkzeugmittelpunkts berechnet wird, und dass der Kalibrierungsparameter die Abweichungen der prognostizierten Position und/oder Orientierung von der bildgestützten Position und/oder Orientierung umfasst. Werden Position und/oder Orientierung des Werkzeugmittelpunkts als Vektor dargestellt, kann die Abweichung insbesondere als komponentenweise Differenz der prognostizierte Position und/oder Orientierung des Werkzeugmittelpunkts und der bildgestützten Position und/oder Orientierung des Werkzeugmittelpunkts bestimmt werden. Die Erfinder haben erkannt, dass die Abweichung der prognostizierten Position und/oder Orientierung von der bildgestützten Position und/oder Orientierung diejenige Größe ist, auf deren Grundlage am schnellsten und präzisesten die Kalibration des Roboters und/oder des Roboterwerkzeugs durchgeführt werden kann.

Nach einem weiteren möglichen Aspekt der Erfindung umfasst der Roboterdatensatz weiterhin einer Geschwindigkeit und/oder eine Beschleunigung der wenigstens einen beweglichen Achse des Roboters während der Aufnahme des Bilddatensatzes, weiterhin basiert das zweite Berechnen des Kalibrierungsparameters derart auf dem Roboterdatensatz, dass aus dem Roboterdatensatz weiterhin eine Geschwindigkeit und/oder eine Beschleunigung und/oder eine Winkelgeschwindigkeit und/oder eine Winkelbeschleunigung des Werkzeugmittelpunkts berechnet wird, und dass der Kalibrierungsparameter weiterhin auf der Geschwindigkeit und/oder auf der Beschleunigung und/oder auf der Winkelgeschwindigkeit und/oder auf der Winkelbeschleunigung des Werkzeugmittelpunkts basiert. Die Erfinder haben erkannt, dass durch die Verwendung von Geschwindigkeits-und/oder Beschleunigungsdaten die Präzision der Kalibrierung verbessert werden kann, insbesondere wenn ein zeitlicher Versatz zwischen der Messung der Roboterdaten und der Aufnahme des Bilddatensatzes vorliegt, oder wenn das zweite Berechnen des Kalibrierungsparameters während einer Bewegung des Roboterwerkzeugs durchgeführt werden muss.

Nach einem weiteren Aspekt der Erfindung umfasst der Roboterdatensatz weiterhin eine Einflussgröße einer Umgebung des Roboters, wobei die Einflussgröße eine Messung des Roboterdatensatzes und/oder die Position des Werkzeugmittelpunkts beeinflusst. Die Erfinder haben erkannt, dass man durch Berücksichtigung einer Einflussgröße einer Umgebung des Roboters die Kalibrierung besonders gut an verschiedene Gegebenheiten der Umgebung anpassen kann.

Nach einem weiteren Aspekt der Erfindung umfasst die Einflussgröße wenigstens die Umgebungstemperatur, die auf das Roboterwerkzeug wirkende Kraft oder die Beladung des Roboterendwerkzeugs. Die Erfinder haben erkannt, dass durch Einbeziehen der Umgebungstemperatur die Ausdehnung die temperaturbedingt unterschiedlichen Ausdehnungen des Roboterwerkzeugs bei der Berechnung des Kalibrierungsparameters berücksichtigt werden können. Weiterhin kann durch das Einbeziehen der auf das Roboterwerkzeug wirkende Kraft oder der Beladung des Roboterwerkzeugs eine Verformung des Roboterwerkzeugs bei der Berechnung des Kalibrierungsparameters berücksichtigt werden.

Nach einem weiteren möglichen Aspekt der Erfindung kann der Roboterdatensatz weiterhin einen Betriebsparameter des Roboterwerkzeugs umfassen, weiterhin kann dann basierend auf dem Betriebsparameter eine binäre Kalibrierungsaufforderung bestimmt werden. Ein Betriebsparameter kann dabei insbesondere die Betriebsstunden des Roboterwerkzeugs oder das Alter des Roboterwerkzeugs betreffen. Die Erfinder haben erkannt, dass basierend auf dem Betriebsparameter besonders schnell und einfach entschieden werden kann, ob eine Kalibrierung notwendig ist. Durch die binäre Kalibrierungsaufforderung kann eine Bedienperson insbesondere feststellen, dass eine Kalibrierung notwendig ist und entsprechende Maßnahmen einleiten. Hierdurch ist es möglich, eine Kalibrierung nur dann durchzuführen, wenn sie tatsächlich notwendig ist.

Weiterhin betrifft die Erfindung eine Kalibrierungseinheit zum Berechnen eines Kalibrierungsparameters eines Roboterwerkzeugs, umfassend folgende Einheiten:
- erste Schnittstelle, ausgebildet zum ersten eines Bilddatensatzes einer medizinischen Bildgebung eines Bildvolumens, wobei die medizinische Bildgebung eine Aufnahme des Bildvolumens mit einem Magnetresonanztomographen, einem Computertomographen, einem Ultraschallgerät und/oder einem Röntgengerät ist,
   wobei das Bildvolumen einen Teil des Roboterwerkzeugs umfasst, und wobei das Roboterwerkzeug an einem Roboter befestigt ist,
- zweite Schnittstelle, ausgebildet zum zweiten Empfangen eines Roboterdatensatzes, wobei der Roboterdatensatz eine Position wenigstens einer beweglichen Achse des Roboters während der Aufnahme des Bilddatensatzes umfasst,
- Recheneinheit, ausgebildet zum Bestimmen der Position und/oder Orientierung einer Markierung im Bilddatensatz, wobei das Roboterwerkzeug die Markierung umfasst, weiterhin ausgebildet zum ersten Berechnen einer bildgestützten Position und/oder Orientierung des Werkzeugmittelpunkts des Roboterwerkzeugs durch Transformieren der Position und/oder Orientierung der Markierung,
   weiterhin ausgebildet zum zweiten Berechnen des Kalibrierungsparameters basierend auf dem Roboterdatensatz sowie auf der bildgestützten Position und/oder Orientierung des Werkzeugmittelpunkts
   wobei aus dem Roboterdatensatz eine prognostizierte Position und/oder Orientierung des Werkzeugmittelpunkts (354) berechnet wird,
   und wobei der Kalibrierungsparameter die Abweichungen der prognostizierten Position und/oder Orientierung von der bildgestützten Position und/oder Orientierung (355) umfasst. Eine solche Kalibrierungseinheit kann insbesondere dazu ausgebildet sein die zuvor beschriebenen erfindungsgemäßen Verfahren und ihre Aspekte auszuführen. Die Kalibrierungseinheit ist dazu ausgebildet diese Verfahren und ihre Aspekte auszuführen, indem die erste Schnittstelle, die zweite Schnittstelle und die Recheneinheit dazu ausgebildet sind, die entsprechenden Verfahrensschritte auszuführen. Die Erfindung betrifft weiterhin einen Roboter umfassend eine erfindungsgemäße Kalibrierungseinheit.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Steuereinrichtungen auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Ein Roboter ist eine universell programmierbare Maschine insbesondere zur Handhabung und zum Bearbeiten von Objekten. Im medizinischen Umfeld können Roboter dazu eingesetzt werden, chirurgische oder bildgebende Werkzeuge an oder in einem menschlichen Körper präzise zu bewegen und einzusetzen. Ein Roboter umfasst eine Bewegungseinheit, ein Roboterwerkzeug und eine Robotersteuerung. Dabei umfasst die Bewegungseinheit mehrere Achsen, die über Verbindungselemente mechanisch gekoppelt sind. Als geläufiger Fachbegriff für Roboterwerkzeug wird auch "Effektor" verwendet. Das Werkzeug kann insbesondere austauschbar ausgebildet sein, damit kann der Roboter dazu ausgelegt sein, mit unterschiedlichen Werkzeugen verwendet zu werden. Bei einer Achse kann es sich insbesondere um eine Linearachse oder um eine Rotationsachse handeln, eine Achse kann insbesondere mit einem Motor angetrieben werden. Die Steuerung ist insbesondere dazu ausgebildet, die Antriebe der Achsen derart zu steuern, dass das Werkzeug eine vorgegebene Position und/oder Orientierung einnimmt.

Ein an einem Roboter angebrachtes Roboterwerkzeug dient im medizinischen Umfeld insbesondere dazu, ein Objekt zu halten und zu positionieren, oder eine Manipulation an einem Objekt durchzuführen. Dabei kann es sich beim Objekt beispielsweise um ein Implantat oder um den Patienten handeln. Ein Roboterwerkzeug kann auch als Plattform ausgebildet sein, an der weitere Roboterwerkzeuge befestigt werden können.

Der Werkzeugmittelpunkt eines Roboterwerkzeugs bezeichnet einen ausgezeichneten Punkt des Roboterwerkzeugs, insbesondere den aktiven Punkt des Roboterwerkzeugs, insbesondere den Punkt, der mit dem zu bearbeitenden Material oder Gewebe in Kontakt ist. Dabei weist der Werkzeugmittelpunkt eine Position und eine Orientierung auf, wobei sowohl die Position als auch die Orientierung vom Roboterwerkzeug vorgegeben sind.

Eine medizinische Bildgebung ist eine Aufnahme eines Bildvolumens insbesondere mit einem Magnetresonanztomographen, einem Computertomographen, einem Ultraschallgerät und/oder einem Röntgengerät. Diese bildgebenden Geräte sind aus dem Stand der Technik bekannt und werden daher hier nicht im Detail beschrieben. Das Bildvolumen ist derjenige Bereich des Raums, der mit der medizinischen Bildgebung dargestellt werden kann. Dabei kann das Bildvolumen insbesondere einen Patienten, einen Teil eines Patienten und/oder ein Roboterwerkzeug umfassen. Mit einem Röntgengerät kann insbesondere eine Röntgenprojektion des Bildvolumens bezüglich einer Projektionsrichtung aufgenommen werden. Ein Bilddatensatz umfasst wenigstens ein Bild, das mit einer medizinischen Bildgebung aufgenommen wurde.

Eine Markierung ist ein Objekt, das mit einer medizinischen Bildgebung derart dargestellt werden kann, dass mittels der medizinischen Bildgebung eine bildgestützte Position und/oder Orientierung der Markierung bestimmt werden kann. Insbesondere umfasst das Roboterwerkzeug die Markierung. Bei einer Markierung kann es sich insbesondere um die Struktur des Roboterwerkzeugs handeln, aber auch um einen Teil des Roboterwerkzeugs, das besonders gut mittels der medizinischen Bildgebung dargestellt werden kann, beispielsweise um eine Anordnung von Bleikugeln in einer medizinischen Bildgebung mit Röntgenstrahlen. Die Struktur des Roboterwerkzeugs kann insbesondere die geometrische Form der Oberfläche umfassen, weiterhin kann die Struktur auch die innere Struktur des Roboterwerkzeugs betreffen.

Die Position eines Objektes bezeichnet eine dreidimensionale Position bezüglich eines Koordinatensystems. Die Orientierung eines Objekts bezeichnet die Orientierung bezüglich eines Koordinatensystems. Das Koordinatensystem kann hierbei bezüglich des Anwendungsraums definiert sein, in dem sich der Roboter befindet, es kann aber auch bezüglich einer Komponente des Roboters definiert sein, wobei die Komponente gegenüber dem Anwendungsraum beweglich ist. Bei dem Koordinatensystem kann es sich insbesondere um ein karthesisches Koordinatensystem handeln. Für Position und Orientierung eines Objekts ist auch der Fachbegriff Pose bekannt. Sowohl Position als auch Orientierung können durch einen Vektor dargestellt werden, insbesondere durch einen dreidimensionalen Vektor.

Im Folgenden wird die Erfindung anhand der in den Figuren dargestellten Ausführungsbeispiele näher beschrieben und erläutert.

Es zeigen
- Fig. 1: ein Flussdiagramm eines Verfahrens zum Berechnen eines Kalibrierungsparameters,
- Fig. 2: eine Kalibrierungseinheit,
- Fig. 3: ein bildgebendes Gerät, einen Roboter und eine Kalibrierungseinheit,
- Fig. 4: ein Roboterwerkzeug.

Die hier gezeigten Kalibrierungseinheiten sowie der hier gezeigte Roboter sind dazu ausgelegt, ein erfindungsgemäßes Verfahren auszuführen.

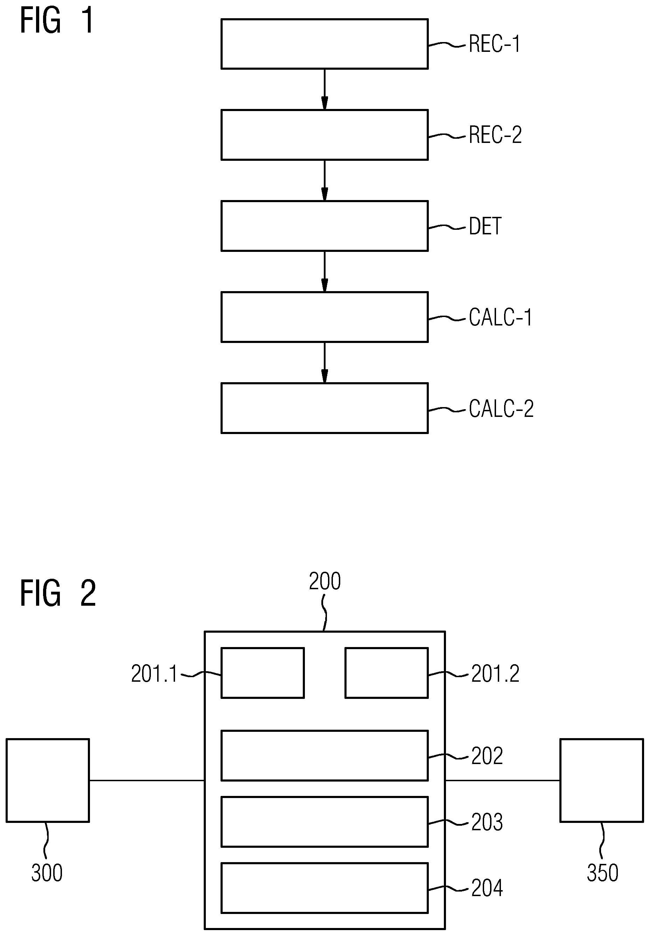

Ein Kalibrierungsparameter kann mit dem in Fig. 1 dargestellten Verfahren berechnet werden. Das Verfahren umfasst ein erstes Empfangen REC-1 eines Bilddatensatzes einer medizinischen Bildgebung eines Bildvolumens mittels einer ersten Schnittstelle 201.1. Herbei umfasst das Bildvolumen wenigstens einen Teil des Roboterwerkzeugs 353. Der Bilddatensatz wird hierbei mit einem bildgebenden Gerät 300 aufgezeichnet, wobei das bildgebende Gerät 300 in diesem Ausführungsbeispiel ein C-Bogen-Röntgengerät 300 ist, mit dem Röntgenprojektionen eines Bildvolumens aus verschiedenen Projektionsrichtungen aufgenommen werden können. Im gezeigten Ausführungsbeispiel werden hierbei mehrere Röntgenprojektionen bezüglich unterschiedlicher Projektionsrichtungen aufgenommen, aus denen ein dreidimensionales Volumenbild rekonstruiert wird. Durch Verwendung von Röntgenprojektionen ist es in diesem Ausführungsbeispiel möglich, die innere Struktur des Roboterwerkzeugs 353 im Bilddatensatz darzustellen. Dadurch kann neben der äußeren Form insbesondere auch die innere Struktur des Roboterwerkzeugs 353 als Markierung dienen. Weiterhin können sich separate Markierungselemente 356.1, 356.2, 356.3 im Inneren des Roboterwerkzeugs befinden, die dazu ausgebildet sind, in der Röntgenbildgebung dargestellt zu werden.

Weiterhin umfasst das in Fig. 1 dargestellte Verfahren ein zweites Empfangen REC-2 eines Roboterdatensatzes mittels einer zweiten Schnittstelle 201.2, wobei der Roboterdatensatz eine Position wenigstens einer beweglichen Achse 351.1, 351.2, 351.3 des Roboters während der Aufnahme des Bilddatensatzes umfasst. Bei einer beweglichen Achse 351.1, 351.2, 351.3 kann es sich um eine Rotationachse handeln, in diesem Fall umfasst die Position der beweglichen Rotationsachse 351.1, 351.2, 351.3 den Winkel zwischen den mittels der Rotationsachse verbundenen Bauteilen. Bei einer beweglichen Achse 351.1, 351.2, 351.3 kann aber auch um eine Linearachse handeln, in diesem Fall umfasst die Position der beweglichen Linearachse 351.1, 351.2, 351.3 die eingestellte Länge der Linearachse. Im gezeigten Ausführungsbeispiel des Verfahrens umfasst der Roboterdatensatz die Position aller beweglichen Achsen 351.1, 351.2, 351.3 des Roboters. Weiterhin umfasst der Roboterdatensatz in diesem Ausführungsbeispiel die Umgebungstemperatur während der bildgebenden Aufnahme, der Roboterdatensatz kann aber auch noch andere Einflussgrößen umfassen, beispielsweise die durch eine Beladung auf das Roboterwerkzeug wirkende Kraft.

Das in Fig. 1 dargestellte Verfahren umfasst weiterhin ein Bestimmen DET der Position und/oder Orientierung einer Markierung im Bilddatensatz mittels der Recheneinheit 202. Im gezeigten Ausführungsbeispiel ist die Markierung durch die äußere Form und die innere Struktur des Roboterwerkzeugs 353 ausgebildet. Weiterhin umfasst die Markierung eine Anordnung von Markierungselementen 356.1, 356.2, 356.3 im Inneren des Roboterwerkzeugs 353. Die Markierung kann aber auch jeweils nur durch die äußere Form des Roboterwerkzeugs 353, die innere Struktur des Roboterwerkzeugs 353 oder durch eine Anordnung von Markierungselementen 356.1, 356.2, 356.3 ausgebildet sein. Die Markierungselemente 356.1, 356.2, 356.3 befinden sich im gezeigten Ausführungsbeispiel im Inneren des Roboterwerkzeugs 353. Die Markierungselemente 356.1, 356.2, 356.3 können sich aber auch außen am Roboterwerkzeug 353 befinden. Im gezeigten Ausführungsbeispiel sind die Markierungselemente durch Bleikugeln ausgebildet, die durch ihre Anordnung die Position und/oder Orientierung der Markierung bestimmen. Es ist auch denkbar, verschieden große Bleikugeln oder Bleiobjekte mit einer anderen Form zu verwenden, so dass ein einzelnes Markierungselement bereits durch seine Form und Ausrichtung die Position und/oder Orientierung der Markierung bestimmt. Es ist ebenfalls denkbar, andere Materialien als Blei zu verwenden, insbesondere solche Materialien, die Röntgenstrahlen stärker absorbieren als die anderen für die Konstruktion des Roboterwerkzeugs 353 verwendeten Materialien.

Weiterhin umfasst das in Fig. 1 dargestellte Verfahren ein erster Berechnen CALC-1 einer bildgestützten Position und/oder Orientierung 355 des Werkzeugmittelpunkts 354 des Roboterwerkzeugs 353 durch Transformieren der Position und/oder Orientierung der Markierung mittels der Recheneinheit 202. Im gezeigten Ausführungsbeispiel ist die Markierung sowohl als Struktur des Roboterwerkzeugs 353 als auch als zusätzliche Anordnung von Markierungselementen 356.1, 356.2, 356.3 ausgebildet. Zur Bestimmung von Position und/oder Orientierung der Markierung werden hierbei im dreidimensionalen Bilddatensatz die Positionen der Markierungselemente 356.1, 356.2, 356.3 bestimmt. Weiterhin werden Positionen von markanten Punkten der Struktur des Roboterwerkzeugs 353 bestimmt, beispielsweise des Endpunkts des Auslegers 359 oder der Schrauben 358 im Befestigungselement 357. Dabei sind die relativen Positionen der Markierungselemente 356.1, 356.2, 356.3 sowie der markanten Punkten der Struktur des Roboterwerkzeugs 353 bekannt, beispielsweise in Form eines dreidimensionalen CAD-Modells des Roboterwerkzeugs. Die Position der Markierungselemente 356.1, 356.2, 356.3 sowie der markanten Punkten der Struktur des Roboterwerkzeugs 353 können daher mit dem CAD-Modell registriert werden, hiermit kann dann die Position und/oder Orientierung 355 des Werkzeugmittelpunkts 354 des Roboterwerkzeugs 353 bestimmt werden. Dabei kann das CAD-Modell durch die im Roboterdatensatz enthaltene Umgebungstemperatur angepasst werden, indem der Wärmeausdehnungskoeffizient der im Roboterwerkzeug 353 verbauten Materialien berücksichtigt wird.

Das in Fig. 1 dargestellte Verfahren umfasst weiterhin ein zweites Berechnen CALC-2 eines Kalibrierungsparameters basierend auf dem Roboterdatensatz sowie auf der bildgestützten Position und/oder Orientierung 355 des Werkzeugmittelpunkts 354 mittels der Recheneinheit 202. Dafür wird aus dem Roboterdatensatz eine vorhergesagte Position und/oder Orientierung des Roboterwerkzeugs 353 und damit eine vorhergesagte Position und/oder Orientierung des Werkzeugmittelpunkts 354 berechnet, indem von einer bekannten Position und Orientierung des Roboterstativs 363 ausgehend über die gemessene Position der beweglichen Achsen 351.1, 351.2, 351.3 und den bekannten Maßen des Roboters 350 die Position und/oder Orientierung der Werkzeughalterung 352 bestimmt wird, und anschließend die bekannten Maße des Roboterwerkzeugs 353 berücksichtigt werden. Die vorhergesagte Position und/oder Orientierung des Werkzeugmittelpunkts 354 wird dann mit der bildgestützten, tatsächlichen Position und/oder Orientierung 355 des Werkzeugmittelpunkts 354 verglichen, indem die komponentenweise Differenz der Positionen und/oder die komponentenweise Differenz der Orientierung als Kalibrierungsparameter bestimmt wird. Mittels dieses Kalibrierungsparameters kann die Robotersteuereinheit 362 die Steuerung der beweglichen Achsen 351.1, 351.2, 351.3 anpassen, beispielsweise indem um die komponentenweise Differenz der Position und/oder Orientierung nachgesteuert wird. Je nach Bauform des Roboters kann es auch notwendig sein, die Kalibrierung anhand von mehreren eingenommener Positionen und/oder Orientierungen des Roboterwerkzeugs durchzuführen, insbesondere wenn mehrere Roboterdatensätze der gleichen vorhergesagten Position und/oder Orientierung entsprechen, beispielsweise bei redundanten Achsen.

Die Schritte des in der Fig. 1 dargestellten Verfahrens können auch in einer anderen als der beschriebenen Reihenfolge ablaufen. Beispielsweise kann das zweite Empfangen REC-2 vor dem ersten Empfangen REC-1 erfolgen, weiterhin das Bestimmen DET der bildgestützten Position kann vor dem zweiten Empfangen REC-2 erfolgen. Auch andere Reihenfolgen der einzelnen Verfahrensschritte sind möglich.

Fig. 2 zeigt eine Kalibrierungseinheit 200 zur Berechnung von Kalibrierungsparametern. Die Kalibrierungseinheit 200 umfasst eine erste Schnittstelle 201.1, eine zweite Schnittstelle 201.2, eine Recheneinheit 202, eine Speichereinheit 203 sowie eine Ein- und Ausgabeeinheit 204 verbunden. Die Kalibrierungseinheit 200 ist direkt mit einem bildgebenden Gerät 300 sowie einem medizinischen Roboter 350 verbunden. Die Verbindung zum bildgebenden Gerät und/oder zum medizinischen Roboter könnte aber auch mittels eines Netzwerks hergestellt werden, beispielsweise ein Intranet oder das Internet. Die hier gezeigte Kalibrierungseinheit 200 ist dazu ausgebildet, das in Fig. 1 dargestellte Verfahren auszuführen. Dabei ist die erste Schnittstelle 201.1 für erste Empfangen REC-1 eines Bilddatensatzes von dem bildgebenden Gerät 300 ausgebildet, die zweite Schnittstelle 201.2 ist für das zweite Empfangen REC-2 eines Roboterdatensatzes von dem medizinischen Roboter ausgebildet, und die Recheneinheit 202 ist für das Bestimmen DET einer der Position und/oder Orientierung einer Markierung im Bilddatensatz, für das erster Berechnen CALC-1 einer bildgestützten Position und/oder Orientierung des Werkzeugmittelpunkts des Roboterwerkzeugs sowie das zweites Berechnen CALC-2 eines Kalibrierungsparameters ausgebildet.

Bei der Kalibrierungseinheit 200 kann es sich insbesondere um einen Computer, einen Mikrocontroller oder um einen integrierten Schaltkreis handeln. Bei der ersten Schnittstellen 201.1 und bei der zweiten Schnittstelle 201.2 kann es sich um eine Hardware- oder Softwareschnittstelle handeln (beispielsweise PCI-Bus, USB oder Firewire). Die erste Schnittstelle 201.1 kann auch mit der zweiten Schnittstelle 201.2 identisch sein. Eine Recheneinheit 202 kann Hardware-Element oder Software-Elemente aufweisen, beispielsweise einen Mikroprozessor oder ein sogenanntes FPGA (englisches Akronym für "Field Programmable Gate Array"). Eine Speichereinheit 203 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Die Speichereinheit 203 kann dazu verwendet werden, den Kalibrierungsparameter zu speichern. Eine Ein- und Ausgabeeinheit 204 umfasst wenigstens eine Eingabeeinheit und/oder wenigstens eine Ausgabeeinheit. Im gezeigten Ausführungsbeispiel kann die Ein- und Ausgabeeinheit dazu verwendet werden, das Verfahren zum Berechnen des Kalibrierungsparameters zu starten und/oder eine Meldung über die erfolgte Berechnung auszugeben. Im hier gezeigten Ausführungsbeispiel ist die Kalibrierungseinheit 200 separat vom medizinischen Roboter 350 ausgebildet. Die Kalibrierungseinheit 200 kann aber auch Teil des medizinischen Roboters 350 oder der Robotersteuereinheit 362 sein, sie kann auch identisch mit der Robotersteuereinheit 362 sein. Alternativ kann die Kalibrierungseinheit 200 auch Teil des bildgebenden Geräts 300 oder der Steuer- und Auswerteeinheit 305 des bildgebenden Geräts 300 sein.

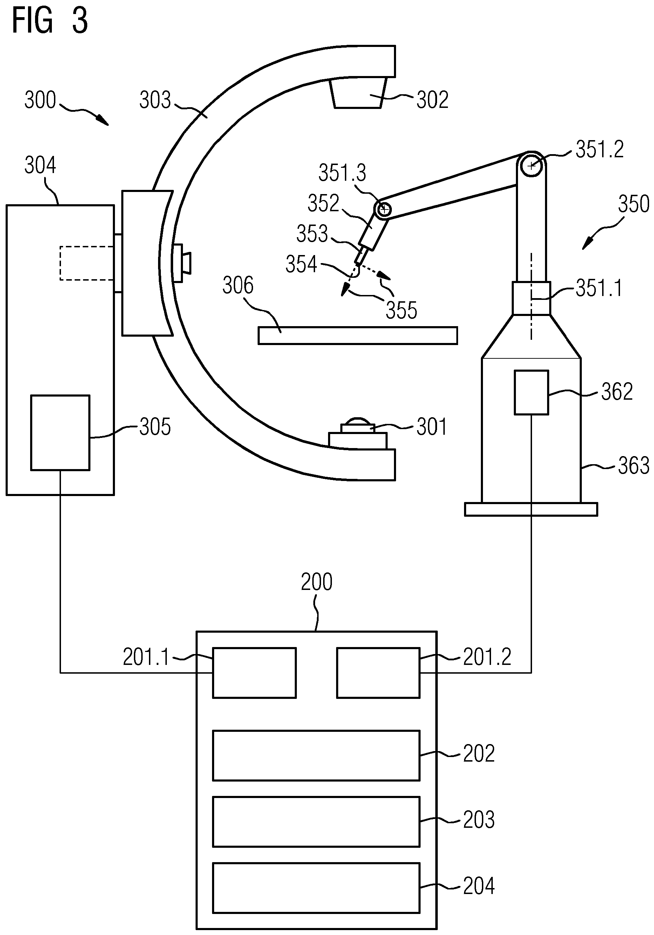

Fig. 3 zeigt ein C-Bogen-Röntgengerät 300, einen medizinischen Roboter 350 sowie eine Kalibrierungseinheit 200. Das C-Bogen-Röntgengerät 300 umfasst eine Röntgenquelle 301 zum Aussenden von Röntgenstrahlen. Weiterhin umfasst das C-Bogen-Röntgengerät 300 einen Röntgendetektor 302 zum Empfangen von Röntgenstrahlen. Die Röntgenquelle 301 sowie der Röntgendetektor 302 sind an den zwei unterschiedlichen Enden des C-Bogens 303 befestigt. Der C-Bogen 303 des C-Bogen-Röntgengeräts 300 ist an einem Stativ 304 befestigt. Das Stativ 304 umfasst Antriebselemente, die dazu ausgelegt sind, die Position des C-Bogens 303 zu verändern. Insbesondere kann der C-Bogen 303 um zwei verschiedene Achsen gedreht werden. Das C-Bogen-Röntgengerät umfasst weiterhin eine Steuer- und Auswerteeinheit 305 sowie eine Patientenlagerungsvorrichtung 306. Mittels der Steuer- und Auswerteeinheit 305 kann die Position des C-Bogens 303 eingestellt werden. Weiterhin können mittels der Steuer- und Auswerteeinheit 305 zweidimensionale Röntgenprojektionen und/oder dreidimensionale Röntgentomographien des Untersuchungsbereichs aus den Rohdaten des Röntgendetektors 302 rekonstruiert werden.

Fig. 3 zeigt weiterhin einen medizinischen Roboter 350. Der medizinische Roboter 350 weist bewegliche Achsen 351.1, 351.2, 351.3 auf, die im gezeigten Ausführungsbeispiel als Rotationsachsen ausgebildet sind. Die beweglichen Achsen 351.1, 351.2, 351.3 können aber auch als Linearachsen ausgebildet sein. Der medizinische Roboter 350 kann weitere bewegliche Achsen aufweisen, die aus Gründen der Übersichtlichkeit in Fig. 3 nicht dargestellt sind. Die beweglichen Achsen 351.1, 351.2, 351.3 sind dazu ausgebildet, eine Werkzeugaufnahme 352 und/oder ein Roboterwerkzeug 353 frei im Untersuchungsbereich zu positionieren. Insbesondere kann die Position und/oder Orientierung 355 des Werkzeugmittelpunkts 354 durch Änderung der Position der beweglichen Achsen 351.1, 351.2, 351.3 verändert werden. Der medizinische Roboter 350 umfasst weiterhin eine Robotersteuereinheit 362 sowie ein Stativ 363. Die Robotersteuereinheit 362 ist insbesondere dazu ausgebildet, die Position der beweglichen Achsen 351.1, 351.2, 351.3 derart einzustellen, dass die Position und/oder Orientierung 355 des Werkzeugmittelpunkts 354 einer vorgegebenen Position und/oder Orientierung entsprechen. Im gezeigten Ausführungsbeispiel ist das Stativ 363 fest im Raum positioniert, das Stativ 363 kann aber auch fahrbar ausgebildet sein.

Im gezeigten Ausführungsbeispiel ist die erste Schnittstelle 201.1 der Kalibrierungseinheit 200 mit der Steuer- und Auswerteeinheit 305 des C-Bogen-Röntgengeräts 300 verbunden. Weiterhin ist die zweite Schnittstelle 201.2 mit der Robotersteuereinheit 362 des medizinischen Roboters 350 verbunden. Es ist natürlich auch möglich, dass das C-Bogen-Röntgengerät 300 eine Schnittstelle aufweist, über die die Kalibrierungseinheit 200 mit der Steuer- und Auswerteeinheit 305 des C-Bogen-Röntgengeräts 300 verbunden ist. Ebenfalls ist es möglich, dass der medizinische Roboter 350 eine Schnittstelle aufweist, über die die Kalibrierungseinheit 200 mit der Robotersteuereinheit 362 verbunden ist.

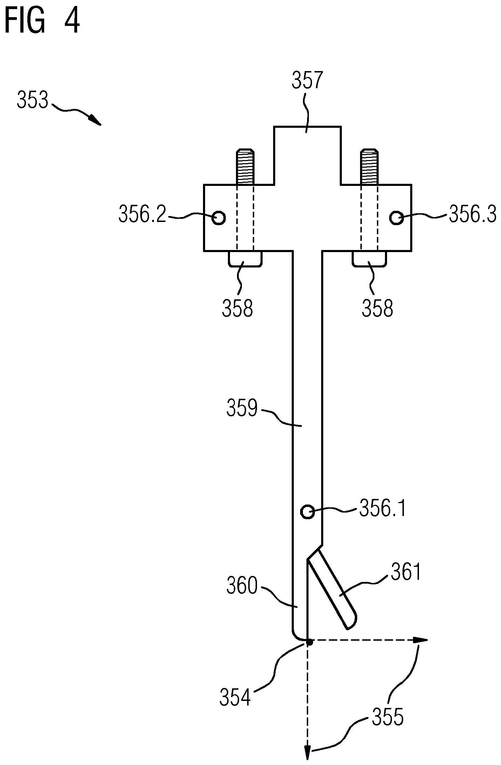

Fig. 4 zeigt ein Roboterwerkzeug 353 zum Greifen und Halten von Objekten. Das Roboterwerkzeug 353 besteht aus einem Befestigungselement 357 sowie einem Ausleger 359. Das Roboterwerkzeug 353 kann mittels des Befestigungselements 357 mit der Werkzeugaufnahme 352 des medizinischen Roboters verbunden werden. Im dargestellten Ausführungsbeispiel kann die Verbindung mittels Schrauben 358 gesichert werden, es sind aber auch andere Verbindungsmittel vorstellbar. An dem von dem Befestigungselement 357 abgewandten Teil des Auslegers 359 ist ein Greifer ausgebildet, der aus einem feststehenden Greifelement 360 sowie einem beweglichen Greifelement 361 besteht. Das bewegliche Greifelement 361 kann beispielsweise direkt mit einem sich im Ausleger 359 befindlichen Motor bewegt werden, oder indirekt mittels eines Seilzuges. Der Werkzeugmittelpunkt 354 des Roboterwerkzeugs 353 ist hier ein Punkt, an dem ein Objekt gehalten werden kann. Die Position des Werkzeugmittelpunkts 354 ist durch die Position des feststehenden Greifelements 360 gegeben, die Orientierung 355 des Werkzeugmittelpunkts 354 ist durch die Vorzugsrichtung des Auslegers 359 und die Bewegungsrichtung des beweglichen Greifelements 361 gegeben.

Beim dargestellten Roboterwerkzeug 353 kann die bildgestützte Position und/oder Orientierung des Roboterwerkzeugs 353 und damit die Position und/oder Orientierung 355 des Werkzeugmittelpunkts im Bilddatensatz bestimmt werden, indem die Struktur des Roboterwerkzeugs 353, insbesondere der Ausleger 259 bzw. das bewegliche Greifelement 361, oder aber auch der Schrauben 358, im Bilddatensatz identifiziert wird. Im gezeigten Ausführungsbeispiel wird das Bestimmen DET der Position und/oder Orientierung des Roboterwerkzeugs 353 erleichtert durch eine Anordnung von Markierungselementen 356.1, 356.2, 356.3. Die Markierungselemente 356.1, 356.2, 356.3 sind hierbei als Bleikugeln ausgebildet. Ein Markierungselement 356.1 befindet sich hierbei innerhalb des Auslegers 359, zwei weitere Markierungselemente 356.2, 356.3 befinden sich innerhalb des Befestigungselements 357.

Um die innere Struktur des Roboterwerkzeugs 353 und damit insbesondere innenliegende Markierungselemente 356.1, 356.2, 356.3 aufzulösen, sollte die Eindringtiefe der Röntgenstrahlung in der Größenordnung der Dicke des Roboterwerkzeugs 353 liegen. Dabei ist die Eindringtiefe abhängig vom Material 353 des Roboterwerkzeugs sowie von der Wellenlänge der verwendeten Röntgenstrahlung. Die Wellenlänge der Röntgenstrahlung kann hierbei beispielsweise durch die Röntgenspannung der Röntgenquelle 301 beeinflusst werden. Wenn die zur Kalibrierung geeignete Wellenlänge größer oder gleich der in der Bildgebung eines Patienten verwendete Wellenlänge, dann kann der Kalibrierungsparameter direkt vor einem Eingriff bestimmt werden. Andernfalls kann der Kalibrierungsparameter auch ohne Patient bestimmt werden.

## Patentansprüche

1. Verfahren zum Berechnen eines Kalibrierungsparameters eines Roboterwerkzeugs (353), umfassend folgende Verfahrensschritte:
- Erstes Empfangen (REC-1) eines Bilddatensatzes einer medizinischen Bildgebung eines Bildvolumens mittels einer ersten Schnittstelle (201.1),
wobei die medizinische Bildgebung eine Aufnahme des Bildvolumens mit einem Magnetresonanztomographen, einem Computertomographen, einem Ultraschallgerät und/oder einem Röntgengerät ist,
wobei das Bildvolumen einen Teil des Roboterwerkzeugs (353) umfasst,
und wobei das Roboterwerkzeug (353) an einem Roboter (350) befestigt ist,
- Zweites Empfangen (REC-2) eines Roboterdatensatzes mittels einer zweiten Schnittstelle (201.2),
wobei der Roboterdatensatz eine Position wenigstens einer beweglichen Achse (351.1, 351.2, 351.3) des Roboters (350) während der Aufnahme des Bilddatensatzes umfasst,
- Bestimmen (DET) der Position und/oder Orientierung einer Markierung im Bilddatensatz mittels einer Recheneinheit (202),
wobei das Roboterwerkzeug (353) die Markierung umfasst,
- Erstes Berechnen (CALC-1) einer bildgestützten Position und/oder Orientierung (355) des Werkzeugmittelpunkts (354) des Roboterwerkzeugs (353) durch Transformieren der Position und/oder Orientierung der Markierung mittels der Recheneinheit (202),
- Zweites Berechnen (CALC-2) des Kalibrierungsparameters basierend auf dem Roboterdatensatz sowie auf der bildgestützten Position und/oder Orientierung (355) des Werkzeugmittelpunkts (354) mittels der Recheneinheit (202),
wobei aus dem Roboterdatensatz eine prognostizierte Position und/oder Orientierung des Werkzeugmittelpunkts (354) berechnet wird,
und wobei der Kalibrierungsparameter die Abweichungen der prognostizierten Position und/oder Orientierung von der bildgestützten Position und/oder Orientierung (355) umfasst.

2. Verfahren nach Anspruch 1, wobei der Bilddatensatz eine zweidimensionale Röntgenprojektion des Bildvolumens umfasst.

3. Verfahren nach einem der vorherigen Ansprüche, wobei der Bilddatensatz ein aus mehreren zweidimensionalen Röntgenprojektionen des Bildvolumens rekonstruiertes dreidimensionales Volumenbild umfasst.

4. Verfahren nach Anspruch 3, wobei das rekonstruierte dreidimensionale Volumenbild aus zweidimensionalen radiographischen Röntgenprojektionen des Bildvolumens rekonstruiert ist.

5. Verfahren nach einem der vorherigen Ansprüche, wobei die Struktur des Roboterwerkzeugs (353) im Bilddatensatz dargestellt ist, und wobei die Markierung als Struktur des Roboterwerkzeugs (353) ausgebildet ist.

6. Verfahren nach Anspruch 5, wobei das erste Berechnen (CALC-1) der bildgestützten Position und/oder Orientierung (355) des Werkzeugmittelpunkts (354) durch Registrieren des Bilddatensatzes mit einem dreidimensionalen Modell des Roboterwerkzeugs (353) erfolgt.

7. Verfahren nach einem der vorherigen Ansprüche, wobei die Markierung eine Mehrzahl von Markierungselementen (356.1, 356.2, 356.3) umfasst, und wobei die Markierungselemente (356.1, 356.2, 356.3) außen am Roboterwerkzeug (353) und/oder innerhalb des Roboterwerkzeugs (353) angeordnet sind.

8. Verfahren nach einem der vorherigen Ansprüche, wobei der Roboterdatensatz weiterhin eine Einflussgröße einer Umgebung des Roboters (350) umfasst, wobei die Einflussgröße eine Messung des Roboterdatensatzes und/oder die Position (355) des Werkzeugmittelpunkts (354) beeinflusst, wobei die Einflussgröße wenigstens eine der folgenden Größen umfasst:
- Umgebungstemperatur,
- auf das Roboterwerkzeug (353) wirkende Kraft,
- Beladung des Roboterwerkzeugs (353),
und wobei bei dem zweiten Berechnen (CALC-2) des Kalibrierungsparameters durch Einbeziehen der Umgebungstemperatur die temperaturbedingt unterschiedlichen Ausdehnungen des Roboterwerkzeugs (353)und/oder durch das Einbeziehen der auf das Roboterwerkzeug (353) wirkende Kraft oder der Beladung des Roboterwerkzeugs (353) eine Verformung des Roboterwerkzeugs (353) berücksichtigt werden.

9. Kalibrierungseinheit (200) zum Berechnen eines Kalibrierungsparameters eines Roboterwerkzeugs (353), umfassend folgende Einheiten:
- erste Schnittstelle (201.1), ausgebildet zum ersten Empfangen (REC-1) eines Bilddatensatzes einer medizinischen Bildgebung eines Bildvolumens,
wobei die medizinische Bildgebung eine Aufnahme des Bildvolumens mit einem Magnetresonanztomographen, einem Computertomographen, einem Ultraschallgerät und/oder einem Röntgengerät ist,
wobei das Bildvolumen einen Teil des Roboterwerkzeugs (353) umfasst, und wobei das Roboterwerkzeug (353) an einem Roboter (350) befestigt ist
- zweite Schnittstelle (201.2), ausgebildet zum zweiten Empfangen (REC-2) eines Roboterdatensatzes,
wobei der Roboterdatensatz eine Position wenigstens einer beweglichen Achse (351.1, 351.2, 351.3) des Roboters (350) während der Aufnahme des Bilddatensatzes umfasst,
- Recheneinheit (202), ausgebildet zum Bestimmen (DET) der Position und/oder Orientierung einer Markierung im Bilddatensatz, wobei das Roboterwerkzeug (353) die Markierung umfasst, weiterhin ausgebildet zum ersten Berechnen (CALC-1) einer bildgestützten Position und/oder Orientierung (355) des Werkzeugmittelpunkts (354) des Roboterwerkzeugs (353) durch Transformieren der Position und/oder Orientierung der Markierung,
weiterhin ausgebildet zum zweiten Berechnen (CALC-2) des Kalibrierungsparameters basierend auf dem Roboterdatensatz sowie auf der bildgestützten Position und/oder Orientierung (355) des Werkzeugmittelpunkts (354)
wobei aus dem Roboterdatensatz eine prognostizierte Position und/oder Orientierung des Werkzeugmittelpunkts (354) berechnet wird,
und wobei der Kalibrierungsparameter die Abweichungen der prognostizierten Position und/oder Orientierung von der bildgestützten Position und/oder Orientierung (355) umfasst.

10. Kalibrierungseinheit (200) nach Anspruch 9, weiterhin ausgebildet ein Verfahren nach einem der Ansprüche 2 bis 8 auszuführen.

11. Computerprogrammprodukt mit einem Computerprogramm, welches direkt in einen Speicher (203) einer Kalibrierungseinheit (200) nach Anspruch 9 oder 10 ladbar ist, mit Programmabschnitten, um alle Schritte des Verfahrens nach einem der Ansprüche 1 bis 8 auszuführen, wenn die Programmabschnitte von der Kalibrierungseinheit (200) ausgeführt werden.

12. Computerlesbares Speichermedium, auf welchem von einer Kalibrierungseinheit (200) nach Anspruch 9 oder 10 lesbare und ausführbare Programmabschnitte gespeichert sind, um alle Schritte des Verfahrens nach einem der Ansprüche 1 bis 8 auszuführen, wenn die Programmabschnitte von der Kalibrierungseinheit (200) ausgeführt werden.

13. Roboter (350), umfassend eine Kalibrierungseinheit (200) nach Anspruch 9 oder 10.

## Claims

1. Method for calculating a calibration parameter for a robot tool (353) comprising the following method steps:
- first reception (REC-1) of an image dataset from a medical imaging of an image volume by means of a first interface (201.1),
wherein the medical imaging is a recording of the image volume with a magnetic resonance tomography scanner, a computed tomography scanner, an ultrasound device and/or an X-ray device,
wherein the image volume comprises a part of the robot tool (353),
and wherein the robot tool (353) is attached to a robot (350),
- second reception (REC-2) of a robot dataset by means of a second interface (201.2),
wherein the robot dataset comprises a position of at least one movable axis (351.1, 351.2, 351.3) of the robot (350) during the recording of the image dataset,
- determination (DET) of the position and/or orientation of a marking in the image dataset by means of a computing unit (202),
wherein the robot tool (353) comprises the marking,
- first calculation (CALC-1) of an image-guided position and/or orientation (355) of the tool centre point (354) of the robot tool (353) by transforming the position and/or orientation of the marking by means of the computing unit (202),
- second calculation (CALC-2) of the calibration parameter based on the robot dataset and on the image-guided position and/or orientation (355) of the tool centre point (354) by means of the computing unit (202),
wherein a predicted position and/or orientation of the tool centre point (354) is calculated from the robot dataset,
and wherein the calibration parameter comprises the deviations of the predicted position and/or orientation from the image-guided position and/or orientation (355).

2. Method according to claim 1, wherein the image dataset comprises a two-dimensional X-ray projection of the image volume.

3. Method according to one of the preceding claims, wherein the image dataset comprises a three-dimensional volume image reconstructed from a plurality of two-dimensional X-ray projections of the image volume.

4. Method according to claim 3, wherein the reconstructed three-dimensional volume image is reconstructed from two-dimensional radiographic X-ray projections of the image volume.

5. Method according to one of the preceding claims, wherein the structure of the robot tool (353) is depicted in the image dataset and wherein the marking is embodied as the structure of the robot tool (353).

6. Method according to claim 5, wherein the first calculation (CALC-1) of the image-guided position and/or orientation (355) of the tool centre point (354) is performed by registering the image dataset with a three-dimensional model of the robot tool (353) .

7. Method according to one of the preceding claims, wherein the marking comprises a plurality of marking elements (356.1, 356.2, 356.3), and wherein the marking elements (356.1, 356.2, 356.3) are arranged on the outside of the robot tool (353) and/or inside the robot tool (353).

8. Method according to one of the preceding claims, wherein the robot dataset furthermore comprises an influencing variable of an environment of the robot (350), wherein the influencing variable influences a measurement of the robot dataset and/or the position (355) of the tool centre point (354), wherein the influencing variable comprises at least one of the following variables:
- ambient temperature,
- force acting on the robot tool (353),
- loading the robot tool (353),
and wherein, in the second calculation (CALC-2) of the calibration parameter, the temperature-induced different extensions of the robot tool (353) are taken into account through the inclusion of the ambient temperature and/or a deformation of the robot tool (353) is taken into account through the inclusion of the force acting on the robot tool (353) or the loading of the robot tool (353).

9. Calibration unit (200) for calculating a calibration parameter for a robot tool (353) comprising the following units:
- a first interface (201.1) embodied for the first reception (REC-1) of an image dataset from a medical imaging of an image volume,
wherein the medical imaging is a recording of the image volume with a magnetic resonance tomography scanner, a computed tomography scanner, an ultrasound device and/or an X-ray device,
wherein the image volume comprises a part of the robot tool (353), and wherein the robot tool (353) is attached to a robot (350),
- a second interface (201.2) embodied for the second reception (REC-2) of a robot dataset,
wherein the robot dataset comprises a position of at least one movable axis (351.1, 351.2, 351.3) of the robot (350) during the recording of the image dataset,
- a computing unit (202) embodied for the determination (DET) of the position and/or orientation of a marking in the image dataset, wherein the robot tool (353) comprises the marking, furthermore embodied for the first calculation (CALC-1) of an image-guided position and/or orientation (355) of the tool centre point (354) of the robot tool (353) by transforming the position and/or orientation of the marking,
furthermore embodied for the second calculation (CALC-2) of the calibration parameter based on the robot dataset and on the image-guided position and/or orientation (355) of the tool centre point (354),
wherein a predicted position and/or orientation of the tool centre point (354) is calculated from the robot dataset,
and wherein the calibration parameter comprises the deviations of the predicted position and/or orientation from the image-guided position and/or orientation (355).

10. Calibration unit (200) according to claim 9, furthermore embodied to carry out a method according to one of claims 2 to 8.

11. Computer program product with a computer program, which can be loaded directly into a memory (203) of a calibration unit (200) according to claim 9 or 10, with program sections for carrying out all the steps of the method according to one of claims 1 to 8 when the program sections are executed by the calibration unit (200).

12. Computer-readable storage medium on which program sections which can be read and executed by a calibration unit (200) according to claim 9 or 10 are stored in order to carry out all the steps of the method according to one of claims 1 to 8 when the program sections are executed by the calibration unit (200) .

13. Robot (350) comprising a calibration unit (200) according to claim 9 or 10.

## Revendications

1. Procédé de calcul d'un paramètre d'étalonnage d'un outil (353) robotisé, comprenant les stades de procédé suivants :
- première réception (REC-1) d'un ensemble de données d'image d'une imagerie médicale d'un volume d'image au moyen d'une première interface (201.1),
l'imagerie médicale étant un enregistrement du volume d'image par un tomodensitomètre à résonnance magnétique, un tomodensitomètre assisté par ordinateur, un appareil à ultrasons et/ou un appareil à rayons X,
le volume d'image comprenant une partie de l'outil (353) robotisé,
et l'outil (353) robotisé étant fixé à un robot (350),
- deuxième réception (REC-2) d'un ensemble de données de robot au moyen d'une deuxième interface (201.2),
l'ensemble de données de robot comprenant une position d'au moins un axe (351.1, 351.2, 351.3) mobile du robot (350) pendant l'enregistrement de l'ensemble de données d'image,
- détermination (DET) de la position et/ou de l'orientation d'un repère dans l'ensemble de données d'image au moyen d'une unité (202) informatique,
dans lequel l'outil (353) robotisé comprend le repère,
- premier calcul (CALC-1) d'une position et/ou d'une orientation (355) s'appuyant sur l'image du centre (354) de l'outil (353) robotisé par transformation de la position et/ou de l'orientation du repère au moyen de l'unité (202) informatique,
- deuxième calcul (CALC-2) du paramètre d'étalonnage sur la base de données de robot, ainsi que de la position et/ou de l'orientation (355) s'appuyant sur l'image du centre (354) de l'outil au moyen de l'unité (202) informatique,
dans lequel, à partir de l'ensemble de données de robot, on calcule une position et/ou une orientation pronostiquée du centre (354) de l'outil,
et dans lequel le paramètre d'étalonnage comprend les écarts de la position et/ou de l'orientation pronostiquée à la position et/ou à l'orientation (355) s'appuyant sur l'image.

2. Procédé suivant la revendication 1, dans lequel l'ensemble de données d'image comprend une projection de rayons X en deux dimensions du volume d'image.

3. Procédé suivant l'une des revendications précédentes, dans lequel l'ensemble de données d'image comprend une image en volume en trois dimensions reconstruite à partir de plusieurs projections de rayons X en deux dimensions du volume d'image.

4. Procédé suivant la revendication 3, dans lequel l'image en volume en trois dimensions reconstruite est reconstruite à partir de projections de rayons X radiographiques en deux dimensions du volume d'image.

5. Procédé suivant l'une des revendications précédentes, dans lequel la structure de l'outil (353) robotisé est représenté dans l'ensemble de données d'image et dans lequel le repère est constitué sous la forme d'une structure de l'outil (353) robotisé.

6. Procédé suivant la revendication (5), dans lequel le premier calcul (CLAC-1) de la position et/ou de l'orientation (355) s'appuyant sur l'image du centre (354) de l'outil s'effectue par enregistrement de l'ensemble de données d'image par un modèle en trois dimensions de l'outil (353) robotisé.

7. Procédé suivant l'une des revendications précédentes, dans lequel le repère comprend une pluralité d'éléments (356.1, 356.2, 356.3) de repérage et dans lequel les éléments (356.1, 356.2, 356.3) de repérage sont disposés à l'extérieur de l'outil (353) robotisé et/ou dans l'outil (353) robotisé.

8. Procédé suivant l'une des revendications précédentes, dans lequel l'ensemble de données de robot comprend, en outre, une grandeur d'influence d'un environnement du robot (350), la grandeur d'influence influençant une mesure de l'ensemble de données de robot et/ou la position (355) du centre (354) de l'outil, la grandeur d'influence comprenant au moins l'une des grandeurs suivantes :
- température ambiante,
- force s'appliquant à l'outil (353) robotisé,
- charge de l'outil (353) robotisé,
et dans lequel, dans le deuxième calcul (CALC-2) du paramètre d'étalonnage, on tient compte, par incorporation de la température ambiante, de dilatations différentes en fonction de la température de l'outil (353) robotisé, et/ou, par incorporation de la force appliquée à l'outil (353) robotisé ou de la charge de l'outil (353) robotisé, d'une déformation de l'outil (353) robotisé.

9. Unité (200) d'étalonnage pour le calcul d'un paramètre d'étalonnage d'un outil (353) robotisé, comprenant les unités suivantes :
- une première interface (201.1), constituée pour la première réception (REC-1) d'un ensemble de données d'image d'une imagerie médicale d'un volume d'image,
l'imagerie médicale étant un enregistrement du volume d'image par un tomodensitomètre à résonnance magnétique, un tomodensitomètre assisté par ordinateur, un appareil à ultrasons et/ou un appareil à rayons X,
le volume d'image comprenant une partie de l'outil (353) robotisé et l'outil (353) robotisé étant fixé à un robot (350),
- une deuxième interface (201.2), constituée pour la réception (REC-2) d'un ensemble de données de robot, l'ensemble de données de robot comprenant une position d'au moins un axe (351.1, 351.2, 351.3) mobile du robot (350) pendant l'enregistrement de l'ensemble de données d'image,
- une unité (202) informatique, constituée pour la détermination (DET) de la position et/ou de l'orientation d'un repère dans l'ensemble de données d'image, l'outil (353) robotisé comprenant le repère, constituée, en outre, pour le premier calcul (CALC-1) d'une position et/ou d'une orientation (355) s'appuyant sur l'image du centre (354) d'outil de l'outil (353) robotisé, par transformation de la position et/ou de l'orientation du repère,
constituée, en outre pour le deuxième calcul (CAL-2) du paramètre d'étalonnage sur la base de l'ensemble de données de robot, ainsi que de la position et/ou de l'orientation (355) s'appuyant sur l'image du centre (354) de l'outil,
dans lequel, à partir de l'ensemble de données de robot, on calcule une position et/ou une orientation pronostiquée du centre (354) de l'outil,
et dans lequel le paramètre d'étalonnage comprend les écarts de la position et/ou de l'orientation pronostiquée à la position et/ou à l'orientation (355) s'appuyant sur l'image.

10. Unité (200) d'étalonnage suivant la revendication 9, constituée, en outre, pour effectuer un procédé suivant l'une des revendications 2 à 8.

11. Produit de programme d'ordinateur, comprenant un programme d'ordinateur, qui peut être chargé directement dans une mémoire (203) d'une unité (200) d'étalonnage suivant la revendication 9 ou 10, comprenant des parties de programme pour effectuer tous les stades du procédé suivant l'une des revendications 1 à 8, lorsque les parties de programme sont réalisées par l'unité (200) d'étalonnage.

12. Support de mémoire déchiffrable par ordinateur, sur lequel sont mises en mémoire des parties de programme pouvant être déchiffrées et réalisées par une unité (200) d'étalonnage suivant la revendication 9 ou 10, afin d'effectuer tous les stades du procédé suivant l'une des revendications 1 à 8, lorsque les parties de programme sont réalisées par l'unité (200) d'étalonnage.

13. Robot (350), comprenant une unité (200) d'étalonnage suivant la revendication 9 ou 10.
